# EUROPÄISCHE PATENTSCHRIFT

(11) **EP 1 304 086 B1**
(45) Veröffentlichungstag und Bekanntmachung des Hinweises auf die Patenterteilung: **29.03.2006**
(21) Anmeldenummer: 02019794.3
(22) Anmeldetag: 05.09.2002
(51) Int. Cl.: A61C 5/06, B05C 17/01, A61M 5/34

(54) **Behältnis für das Aufbringen einer dentalen Masse**
Container for dispensing a dental composition
Emballage pour délivrer une composition dentaire

(30) Priorität: 18.10.2001 DE 10151404
(43) Veröffentlichungstag der Anmeldung: 23.04.2003
(73) Patentinhaber: Firma Ivoclar Vivadent AG, 9494 Schaan (LI)
(72) Erfinder: Reisinger, Michael, 9491 Ruggell (LI)
(74) Vertreter: Baronetzky, Klaus

(56) Entgegenhaltungen:
- EP-A- 0 063 891
- EP-A- 0 919 206
- WO-A-89/12428
- US-A- 4 472 141
- US-A- 4 619 613
- US-A- 4 619 640
- US-A- 5 725 511

## Beschreibung

Die Erfindung betrifft ein Set, bestehend aus einem Behältnis und einer Ausbringdüse oder einer Verschlussklappe gemäß dem Oberbegriff von Anspruch 1.

Derartige Behältnisse, die beispielsweise als Spritzen ausgebildet sein können, sind seit langem bekannt. Bei der Lösung gemäß der EP-A1-63 891 wird eine Ausbringdüse mit einer Verschlusskappe versehen, wobei pastöse Masse in der Ausbringdüse vorgesehen ist. Ein Stempel drückt einen Spritzenkolben in ein Spritzengehäuse hinein, wobei durch eine entsprechende Formgebung des Spritzenkolbens dafür gesorgt werden soll, dass möglichst geringe Reste verbleiben.

Gemäß der genannten Patentveröffentlichung ist eine Verschlusskappe vorgesehen, die farbkodiert ist. Durch die Codierung soll sichergestellt werden, dass die Ausbringdüse und die Verschlusskappe je als zusammengehörend erkannt werden, damit nicht versehentlich eine Verschlusskappe auf die falsche Ausbringdüse aufgebracht wird.

Ferner ist aus der Druckschrift US-A-4 472 141 eine Vorrichtung für Zahnbehandlungen bekannt, die aus einem spritzenartigen Behältnis, einem Druckstempel, einer Spitze sowie einem Handgriff mit auswechselbaren Hebeln, abhängig vom auszubringenden Material und dem dafür nötigeri Vorschub bzw. Druck des Stempels, besteht. Darüberhinaus wird hier ebenfalls eine Farbcodierung des Spritzenbehälters und des zugehörigen Druckstempelendes beschrieben, die ein Verwechseln verhindern sollen.

Hintergrund ist, dass unterschiedliche Massen mit entsprechend farbig gestalteten Ausbringdüsen appliziert werden können. Die verschiedenen pastösen Massen weisen gerade bei Anwendung im Dentalbereich spezielle Eigenschaften auf, die durch Verunreinigungen beeinträchtigt werden. Es ist aber wichtig, dass der Zahnarzt oder Zahntechnilcer sich auf die zugesicherten Eigenschaften der verwendeten Masse verlassen kann.

Die verwendeten Massen sind andererseits häufig photopolymesisierbare Massen. Die regelmäßig vorgesehene Lagerung in einem geschlossenen und lichtdichten Schrank erlaubt es, die aufbewahrte Masse auch nach längerer Zeit ohne Qualitätseinbußen zu verwenden. Wenn andererseits durch die Abhebung der die Masse aufnehmenden Ausbringdüse die Identifizierung vorgenommen werden soll, ist es wichtig, dass die Ausbringdüsen regelmäßig in dem lichtdichten Schrank gelagert werden, denn beispielsweise hat ein gelber Ausbringkörper eine erheblich größere Lichtdurchlässigkeit als ein dunkelblaues Spritzengehäuse. Daher ist gemäß der Patentschrift EP-A1-63 891 ein recht dickwandiges Gehäuse vorgesehen.

Auch die in der Druckschrift US-A-4 472 141 beschriebene Farbcodierung hat den oben beschriebenen Nachteil, dass lichthärtendes Material bei der Aufbewahrung in diesen Behältern und Spitzen, vor allem in hellen Farben, Umgebungslicht ausgesetzt ist, das eine unerwünschte Reaktion des Materials im Behälter in Gang setzen kann.

Zwar ist es möglich, einen zweischichtigen Aufbau für das Spritzengehäuse zu wählen und die innere Schicht aus einem lichtundurchlässigen Material herzustellen. Diese Lösung ist jedoch bedeutend teuerer und erfordert eine höhere Herstellungspräzision zur Abstimmung der Schichtdicken aufeinander, der Verankerung der Schichten aneinander sowie auch der Dichtheit.

Ferner ist aus der Druckschrift WO-A-98/12428 eine spritzenförmige Vorrichtung bekannt, mit der Stoffe zur Zahnbehandlung ausgebracht werden können, die in der zugehörigen röhrenförmigen gekrümmten Spitze verschiebbare Borsten enthält, um den auzubringenden Stoff für die Behandlung wahlweise punktuell oder flächig gleichmäßig verteilt auftragen zu können. Durch die ebenfalls beschriebene Möglichkeit, die Spitzen austauschbar vorzusehen, besteht jedoch auch hier das Risiko, eine Spitze, die vorher bereits mit einem anderen Stoff benetzt wurde, an dem Vorratsbehälter mit einem Stoff zu befestigen, der durch den Rest in der Spitze verunreinigt wird oder mit diesem eine unkontrollierte Reaktion eingeht.

Ein Nachteil der bislang verwendeten Systeme liegt darin begründet, dass die Spritzen zusammen mit der Ausbringdüse hergestellt werden müssen. Zwar können die Spritzen als Wegwerfartikel im Grunde noch recht preiswert hergestellt werden. Problem ist jedoch, dass die viskösitäten der aufzubringenden Massen unterschiedlich sind. Daher wäre es wünschenswert, in Abhängigkeit von der aufzubringenden Masse geeignete Widerstände in der Ausbringdüse vorzusehen. Der Zahnarzt oder Zahntechniker sollte nach Möglichkeit unabhängig von der Viskosität der aufzubringenden Masse eine gleich bleibende Betätigungskraft ausüben können, die in ergonomischer Hinsicht optimiert ist und eine feinfühlige Dosierung erlaubt.

Bei den bekannten Spritzen wird allerdings in Kauf genommen, dass die Betätigungskräfte je nach aufzubringender Masse unterschiedlich sind, nachdem die Herstellung von zahlreichen verschiedenen Spritzen mit entsprechenden Durchmessern nach Viskosität der Masse zu aufwändig wäre, zumal sich die Viskositäten bei der Neuentwicklung von Massen auch ändern. Daher ist man bislang hinsichtlich der Festlegung des Ausbringwiderstands einen Kompromiss eingegangen und hat eine Spritze mit einem mittleren Ausbringwiderstand bereitgestellt.

Andererseits nehmen die Ansprüche an die Dentalmassen noch zu, so dass in Zukunft eher zu erwarten ist, daß die Anzahl der aufzubringenden Massen unterschiedlicher Viskosität noch ansteigt.

Daher legt der Erfindung die Aufgabe zu Grunde, ein Behältnis für das Aufbringen einer insbesondere pastösen Masse für den Dentalbereich zu schaffen, die hinsichtlich der Handhabung deutlich verbessert ist, wobei sich dennoch Einsparungen bei der Herstellung erzielen lassen.

Diese Aufgabe wird erfindungsgemäß durch Anspruch 1 gelöst. Vorteilhafte Weiterbildungen ergeben sich aus den Unteransprüchen.

Erfindungsgemäß ist es vorgesehen, das Gehäuse als Standardprodukt mit unterschiedlichen Ausbringdüsen zu kombinieren, die ebenfalls als handelsübliche Produkte bereitgestellt werden können. Bei Ausgestaltung eines erfindungsgemäßen Behältnis als Spritze sind derartige Systeme mit einer sogenannten Luer-Lock-Verbindung im Handel erhältlich und lassen sich dementsprechend preisgünstig beschaffen. Erfindungsgemäß wird nun auf diese Kombinationen aus Spritzen und Ausbringdüsen je eine geeignete Markierung aufgebracht, die eine Verwechslung ausschließt.

Es versteht sich, dass geeignete und aussagekräftige Markierungen verwendbar sind. Wenn beispielsweise sechs verschiedene Massen in drei unterschiedlichen Viskositäten gehandhabt werden sollen, bietet es sich an, drei geeignete Symbole als Markierungen einzusetzen, wie beispielsweise einen Kreis, ein Quadrat und ein Dreieck. Es versteht sich, dass anstelle dessen auch beliebige andere geeignete Markierungen, Symbole oder dergleichen mit Hinweischarakter verwendet werden können. Beispielsweise können auch die unterschiedlichen Spritzendüsendurchmesser symbolisiert werden, also etwa durch Kreise mit drastisch unterschiedlichen Durchmessern.

Eine weitere Möglichkeit besteht in der Angabe des Auslassdurchmessers in Millimetern.

Besonders günstig ist es, daß mit der erfindungsgemäßen Markierung lichtgeschützte Spritzen und Ausbringdüsen standardmäßig verwendet werden können, während die Markierung nach Farbe und Form wählbar ist.

Es vesteht sich, dass anstelle der vorstehend genannten Luer-Lock-Verbindung eine beliebige andere geeignete Verbindung zwischen dem Spritzengehäuse und der Ausbringdüse möglich ist, ohne den Bereich der Erfindung zu verlassen. So kann beispielsweise ohne Weiteres ein Bajonettverschluss eingesetzt werden, wobei es günstig ist, in an sich bekannter Weise eine ringförmige Dichtung zu verwenden, die das Spritzengehäuse und die Ausbringdüse gegeneinander druckdicht abdichtet.

Besonders günstig ist es ferner, daß die Markierungen bei geeigneter Anbringung zugleich auch eine optische Rückkopplung für die richtige Drehposition bilden. Bevorzugt sind die Markierungen so angebracht, dass sie dann übereinander liegen, wenn die Luer-Lock-Verbindung oder die Bajonettverbindung sicher verbunden sind.

In bevorzugter Ausgestaltung ist die Spritze im Vergleich zum Volumen der Ausbringdüse recht großvolumig. Der Volumenunterschied kann beispielsweise auch 20:1 oder gar 100:1 betragen. Die Spritze nimmt nach der Art eines Vorratsbehälters die Masse auf, während die Ausbringdüse oder Kanüle als Wegwerfteil ausgebildet sein kann.

Bei dieser Ausgestaltung ist es vorgesehen, für das Spritzengehäuse eine Verschlusskappe einzusetzen. Derartige Verschlusskappen sind ebenfalls im Handel für das Luer-Lock-System erhältlich. Bevorzugt wird erfindungsgemäß die Verschlusskappe dann ebenfalls mit einer entsprechenden Markierung versehen, um sicher auszuschließen, daß Verunreinigungen durch Verwechslung der Verschlusskappen zwischen zwei Spritzen entstehen können.

Auch wenn eine unverlierbare Anbringung der Etiketten, beispielsweise durch Aufdruck, durch Prägung, durch abriebfeste Färbung oder dergleichen bevorzugt ist, versteht es sich, daß es grundsätzlich auch möglich ist, die Markierung über Etiketten zu realisieren. Derartige Etiketten bieten auch die Möglichkeit, die Markierungen mit Strichcodierungen zu kombinieren, um die Maschinenlesbarkeit und damit die Automatisierung der Lagerhaltung zu verbessern.

Weitere Vorteile, Einzelheiten und Merkmale ergeben sich aus der nachfolgenden Beschreibung eines Ausführungsbeispiels anhand der Zeichnung.

Es zeigen:
- Fig. 1: eine erfindungsgemäße Spritze mit einer ersten Markierung, in einer schematischen Ansicht;
- Fig. 2: eine erfindungsgemäße Spritze mit einer zweiten Markierung, in einer schematischen Ansicht;
- Fig. 3: eine erfindungsgemäße Spritze mit einer dritten Markierung, in einer schematischen Ansicht;
- Fig. 4: eine Schnittansicht eines vorderen Teils einer erfindungsgemäßen Spritze; und
- Fig. 5: eine schematische Teilansicht eines vorderen Teils der erfindungsgemäßen Spritze in teilweise aufgebrochener Darstellung.

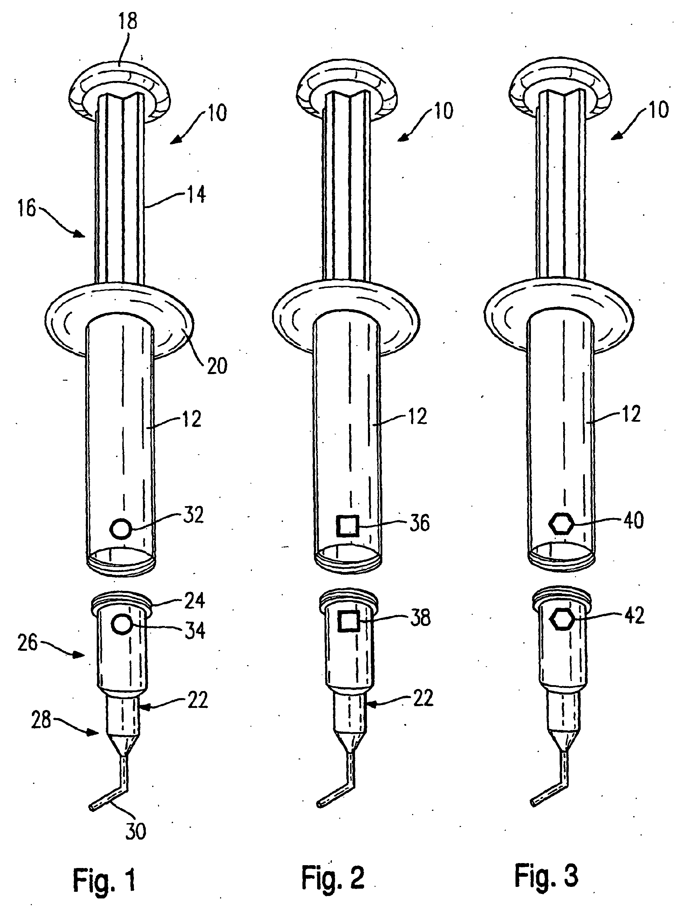

Die in Fig. 1 dargestellte Spritze 10 weist ein Spritzengehäuse 12 auf, das einen Spritzenkolben 14 führt. Von dem Spritzenkolben 14 ist aus den Figuren 1-3 lediglich der Schaft ersichtlich. Zur Erzeugung des erforderlichen Drucks weist der Schaft 16 an seinem Ende einen Knauf 18 auf. Hierzu entsprechend weist das Spritzengehäuse 12 einen Greifflansch 20 auf. Durch Zusammendrücken von Knauf 18 und Greifflansch 20 zwischen dem Handballen und beispielsweise Mittel- und Zeigefinger kann ein recht großer Druck ausgeübt werden, der die in dem Spritzengehäuse 12 aufgenommene pastöse Masse nach vorne drückt.

An das Spritzengehäuse 12 ist vorne eine Ausbringdüse 22 angeschlossen. Hierzu weist das Spritzengehäuse 12 an seinem vorderen Ende ein recht kurzes Innengewinde mit einer vergleichsweise großen Steigung auf. Zu dem Innengewinde passt ein Außengewinde 24 am rückwärtigen Ende der Ausbringdüse 22. Die Passung der Gewinde zueinander ist recht spielarm vorgesehen .

Die Ausbringdüse weist einen zylindrischen Teil 26, einen konischen Teil 28 und eine abgewinkelte Kanüle 30 auf.

Auch wenn das Volumen der Ausbringdüse 22 in den Zeichnungen nur um weniger als eine Größenordnung kleiner ist als das Volumen des Spritzengehäuses 12 dargestellt ist, versteht es sich, daß es in der Praxis wesentlich kleiner sein kann.

Erfindungsgemäß weisen das Spritzengehäuse 12 und die Ausbringdüse 22 je eine Markierung 32 und 34 auf. Beide Markierungen stimmen überein und kommen dann übereinander zu liegen, wenn die Ausbringdüse 22 fest mit dem Spritzengehäuse 12 verbunden ist.

In der dargestellten Ausführungsform sind beide Markierungen als Kreis ausgebildet.

Erfindungsgemäß ist es vorgesehen, für eine andere aufzunehmende Masse eine andere Markierung zu wählen: So ist in Fig. 2 eine Spritze 10 dargestllt, die im übrigen mit der Spritze 10 gemäß Fig. 1 übereinstimmt, jedoch mit einer Markierung 36 am Spritzengehäuse und einer Markierung 38 an der Ausbringdüse 22 versehen ist, die sich deutlich von den Markierungen 32 und 34 unterscheidet.

Entsprechendes gilt für die dritte Ausführungsform der erfindungsgemäßen Spritze 10 gemäß Fig. 3. Dort sind die dortigen Markierungen 40 und 42 als Sechsecke ausgebildet.

Die Markierungen können in beliebiger geeigneter Weise aufgebracht werden. In den dargestellten Ausführungsbeispielen sind sie als entsprechend ausgestanzte Kunststoffteile vorgesehen, die unverlierbar auf dem Spritzengehäuse 12 bzw. der Ausbringdüse 22 befestigt sind.

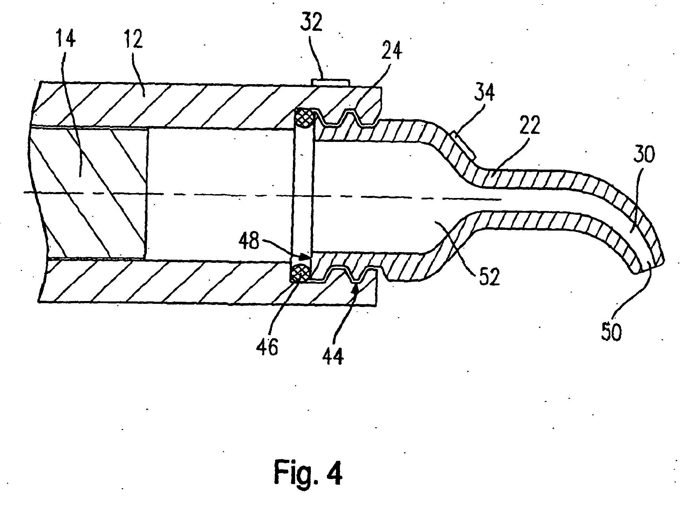

Aus Fig. 4 ist die bevorzugte Verbindung zwischen dem Spritzengehäuse 12 und der Ausbringdüse 22 ersichtlich. Ein Innengewinde 44 führt das Außengewinde 24 der Ausbringdüse 22. Ein Dichtring 46 erstreckt sich zwischen der Stirnfläche 48 der Ausbringdüse 22 und einer Innenecke des Spritzengehäuses 12.

Die Kanüle 30 weist einen Innendurchmesser 50 auf, der genau bestimmt ist und zwischen den Ausbringdüsen mit unterschiedlichen Markierungen unterschiedlich ist. Bei viskoseren Massen 52, die in der Ausbringdüse 22 und dem Spritzengehäuse 12 aufgenommen sind, wird eine größerer Innendurchmesser 50 gewählt, während weniger viskose Massen einen geringeren Innendurchmesser 50 erlauben. Auf diese Art ist die Kraft, die für den Vortrieb des Kolbens 14 erforderlich ist, im Wesentlichen gleich, unabhängig von der Viskosität der Masse 52.

Nach Gebrauch wird die Ausbringdüse 22 verworfen und das Spritzengehäuse 12 mit einer Verschlusskappe zugeschraubt. Die Verschlusskappe ist nicht dargestellt, weist aber ein Außengewinde auf, das sich in das Innengewinde 44 einschrauben läßt. Wenn die Verschlusskappe aufgebracht ist, läßt sich das in dem Spritzengehäuse 12 aufbewahrte Material auch über längere Zeit lagern, ohne auszuhärten.

Sowohl das Spritzengehäuse 12 als auch die Ausbringdüse 22 bestehen aus schwarz eingefärbtem Kunststoff, beispielsweise Polyamid, und sind je einstückig.

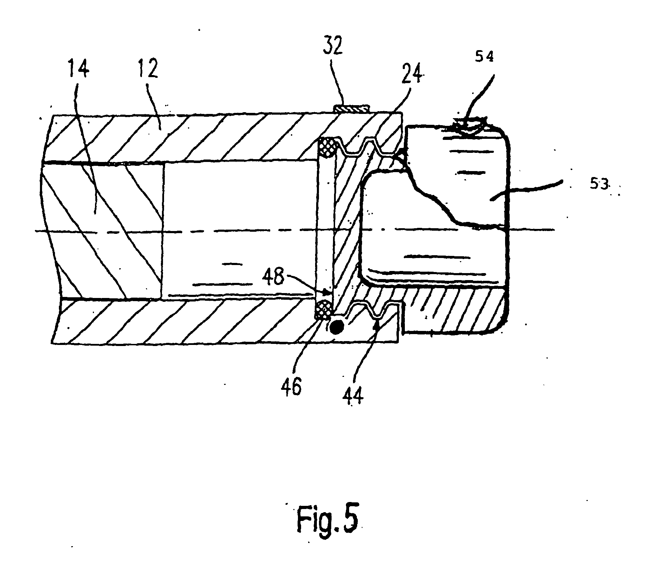

Aus Fig. 5 ist eine Darstellung des vorderen Teils einer erfindungsgemäßen Spritze ersichtlich, wie sie zu Lagerzwecken verwendet werden kann. Die Spritze ist in ihrem vorderen Teil mit einem Verschlussstopfen 53 versehen, der anstelle der Ausbringdüse 22 eingeschraubt ist und als Verschlusskappe dient. Dementsprechend weist der Verschlussstopfen 53 ein Außengewinde auf, das ebenfalls zum Innengewinde 44 des Spritzengehäuses 12 passt.

Der Verschlussstopfen 53 weist ebenfalls eine Markierung 54 auf, die zu der Markierung 32 passt und die Zugehörigkeit zu der betreffenden Spritze symbolisiert.

Zugleich ist durch die Übereinstimmung der horizontalen Drehposition, also das Fluchten der Markierungen 32 und 54, sichergestellt, dass der Verschlussstopfen 53 einerseits fest eingeschraubt ist, aber andererseits das Innengewinde 44 nicht durch den Verschlussstopfen 53 überdreht wird. Damit kann letztlich auch die auf die Dichtung 46 wirkende Anpresskraft präzise eingestellt werden, so dass eine optimale Abdichtung gewährleistet ist.

Die erfindungsgemäße Lösung ist nicht auf die Ausgestaltung des Behältnisses als Spritze beschränkt. Vielmehr kann mit besonderen Vorteilen das Behältnis auch als Tube ausgebildet sein, die pastöse Masse aufnimmt. Ferner kann beispielsweise ein Fläschchen als Behältnis auch eine Flüssigkeit oder ein Pulver aufnehmen.

## Patentansprüche

1. Set, bestehend aus einem Behältnis (12) und einer Ausbringdüse (22) oder einer Verschlusskappe, für das Aufbringen einer, inbesondere pastösen, Masse für den Dentalbereich, die in dem Behältnis (12) gelagert ist, wobei ein Gehäuse des Behältnisses einen Anschluss aufweist, der mit der Ausbringdüse (22) oder der Verschlusskappe verbindbar ist, die druckfest und dicht an dem Gehäuse befestigbar ist und von ihm lösbar ist, **dadurch gekennzeichnet, dass** die Ausbringdüse (22) oder die Verschlusskappe eine Formmarkierung (34, 38, 42) aufweist, die einer Formmarkierung (32, 36, 40) auf dem Gehäuse entspricht.

2. Set nach Anspruch 1, **dadurch gekennzeichnet, dass** der Anschluss ein positionierender Anschluss ist, der bei druckfester Verbindung zwischen Ausbringdüse (22) und Spritzenkörper eine eindeutige Drehposition aufweist und das in dieser Drehposition die Markierung auf der Ausbringdüse (22) unterhalb der Markierung auf dem Gehäuse (12) angeordnet ist.

3. Set nach einem der vorhergehenden Ansprüche, **dadurch gekennzeichnet, dass** der Anschluss in Luer-Lock-Technik ausgebildet ist.

4. Set nach einem der vorhergehenden Ansprüche 1 oder 2, **dadurch gekennzeichnet, dass** der Anschluss in Bajonettverschluss-Technik ausgebildet ist.

5. Set nach einem der vorhergehenden Ansprüche, **dadurch gekennzeichnet, dass** gleiche Markierungen dem gleichen Enddurchmesser der Ausbringdüse (22) und entsprechend verschiedene Markierungen verschiedenen Durchmessern der Ausbringdüse (22) entsprechen.

6. Set nach einem der vorhergehenden Ansprüche, **dadurch gekennzeichnet, dass** die Markierungen als Symbole ausgebildet sind.

7. Set nach einem der vorhergehenden Ansprüche, **dadurch gekennzeichnet, dass** die Markierungen als Zahlen oder Buchstaben ausgebildet sind.

8. Set nach einem der vorhergehenden Ansprüche, **dadurch gekennzeichnet, dass** die Markierungen den Enddurchmesser der Ausbringdüse (22) in Millimetern angeben.

9. Set nach einem der vorhergehenden Ansprüche, **dadurch gekennzeichnet, dass** die Ausbringdüse (22) als Wegwerfteil ausgebildet ist und die Behältnis (10) über die Verschlusskappe verschließbar ist.

10. Set nach einem der vorhergehenden Ansprüche, **dadurch gekennzeichnet, dass** das Behältnis (10) ein Standardbehältnis mit Luer-Lock ist, auf welchem die Markierung aufgebracht ist.

11. Set nach einem der vorhergehenden Ansprüche, **dadurch gekennzeichnet, dass** das Behältnis (10) lichtdicht ausgebildet ist und insbesondere ein schwarzes Gehäuse (12) aufweist.

12. Set nach einem der vorhergehenden Ansprüche, **dadurch gekennzeichnet, dass** das Behältnis (10) als Spritze ausgebildet ist, ein Gehäuse (22) einen Spritzenkolben (14) führt und die Masse (52) aus dem Behältnis (10) bei Druck auf den Spritzenkolben (14) ausbringbar ist.

13. Set nach einem der vorhergehenden Ansprüche, **dadurch gekennzeichnet, dass** das Behältnis (10) als Flasche oder als Tube ausgebildet ist.

14. Set nach einem der verhergehenden Ansprüche, **dadurch gekennzeichnet, dass** die Masse ein Pulver oder eine Flüssigkeit ist.

## Revendications

1. Ensemble, composé d'un récipient (12) et d'une buse d'injection (22) ou d'un capuchon, pour l'application d'une masse, en particulier pâteuse, de dentisterie, qui est contenue dans le récipient (12), dans lequel un corps du récipient présente un embout qui peut être couplé à la buse d'injection (22) ou au capuchon, qui peut être fixé de manière étanche à la pression et hermétiquement sur le corps et qui peut être détaché de ce dernier, **caractérisé en ce que** la buse d'injection (22) ou le capuchon présente un repère géométrique (34, 38, 42) qui correspond à un repère géométrique (32, 36, 40) situé sur le corps.

2. Ensemble selon la revendication 1, **caractérisé en ce que** l'embout est un embout de positionnement, qui indique une position de rotation univoque lors d'un couplage étanche à la pression entre la buse d'injection (22) et le corps d'injection et **en ce que**, dans cette position de rotation, le repère sur la buse d'injection (22) est situé en dessous du repère sur le corps (12).

3. Ensemble selon l'une quelconque des revendications précédentes, **caractérisé en ce que** l'embout est conçu selon la technique Luer-Lock.

4. Ensemble selon l'une quelconque des revendications 1 ou 2, **caractérisé en ce que** l'embout est conçu selon la technique de fixation à baionnette.

5. Ensemble selon l'une quelconque des revendications précédentes, **caractérisé en ce que** des repères identiques correspondent au même diamètre d'extrémité de la buse d'injection (22) et inversement, des repères différents correspondent à des diamètres différents de la buse d'injection (22).

6. Ensemble selon l'une quelconque des revendications précédentes, **caractérisé en ce que** les repères sont conçus sous forme de symboles.

7. Ensemble selon l'une quelconque des revendications précédentes, **caractérisé en ce que** les repères sont conçus sous forme de chiffres ou de lettres.

8. Ensemble selon l'une quelconque des revendications précédentes, **caractérisé en ce que** les repères indiquent le diamètre d'extrémité de la buse d'injection (22) en millimètres.

9. Ensemble selon l'une quelconque des revendications précédentes, **caractérisé en ce que** la buse d'injection (22) est conçue sous forme d'élément jetable et le récipient (10) est refermable à l'aide du capuchon.

10. Ensemble selon l'une quelconque des revendications précédentes, **caractérisé en ce que** le récipient (10) est un récipient classique équipé d'un système Luer-Lock sur lequel est porté le repère.

11. Ensemble selon l'une quelconque des revendications précédentes, **caractérisé en ce que** le récipient (10) est conçu de manière étanche à la lumière et en particulier il présente un corps (12) noir.

12. Ensemble selon l'une quelconque des revendications précédentes, **caractérisé en ce que** le récipient (10) est conçu sous forme de seringue, un corps (22) guide un piston d'injection (14) et la masse (52) peut être éjectée du récipient (10) par pression sur le piston d'injection (14).

13. Ensemble selon l'une quelconque des revendications précédentes, **caractérisée en ce que** le récipient (10) est conçu sous forme de flacon ou de tube.

14. Ensemble selon l'une quelconque des revendications précédentes, **caractérisé en ce que** la masse est une poudre ou un liquide.

## Claims

1. A set comprising a container (12) and an application nozzle (22) or a closure cap for applying an, in particular, pasty compound for use in the dental sector, which is stored in the container (12), wherein a housing of the container has a connection which can be connected to the application nozzle (22) or the closure cap which can be fastened in a pressure-resistant and fluid-tight manner to the housing and can be released therefrom, **characterised in that** the application nozzle (22) or the closure cap has a shaped marking (34,38,42) which corresponds to a shaped marking (32,36,40) on the housing.

2. A set according to Claim 1, **characterised in that** in the connection is a locating connection which with a pressure-resistant connection between the application nozzle (22) and the syringe body has a definite rotational position, and **in that** in this rotational position the marking on the application nozzle (22) is disposed below the marking on the housing (12).

3. A set according to one of the preceding Claims, **characterised in that** the connection is designed using clip-lock technology.

4. A set according to either of the preceding Claims 1 or 2, **characterised in that** in the connection is designed using bayonet lock technology.

5. A set according to any one of the preceding Claims, **characterised in that** equal markings correspond to an equal end diameter of the application nozzle (22) and correspondingly different markings correspond to different diameters of the application nozzle (22).

6. A set according to any one of the preceding Claims, **characterised in that** the markings are in the form of symbols.

7. A set according to any one of the preceding Claims, **characterised in that** the markings are in the form of numerals or letters.

8. A set according to any one of the preceding Claims, **characterised in that** the markings indicate the end diameter of the application nozzle (22) in millimetres.

9. A set according to any one of the preceding Claims, **characterised in that** the application nozzle (22) is in the form of a disposable part and the container (10) can be sealed by way of the closure cap.

10. A set according to any one of the preceding Claims, **characterised in that** the container (10) is a standard container with a clip-lock, on which the marking is applied.

11. A set according to any one of the preceding claims, **characterised in that** the container is of light-proof design and, in particular, has a black housing (12).

12. A set according to any one of the preceding claims, **characterised in that** the container is the form of a syringe, a housing (22) guides a syringe plunger (14) and the compound can be discharged from the container (10) when pressure is applied to the syringe plunger (14).

13. A set according to any one of the preceding claims, **characterised in that** the container (10) is in the form of a cylinder or tube.

14. A set according to any one of the preceding claims, **characterised in that** the compound is a powder or a fluid.
